# EUROPEAN PATENT SPECIFICATION

(11) **EP 4 297 841 B1**
(45) Date of publication and mention of the grant of the patent: **12.02.2025**
(21) Application number: 21712361.1
(22) Date of filing: 23.02.2021
(51) Int. Cl.: A61M 60/117, A61M 60/268, A61M 60/449, A61M 60/835, A61M 60/837, A61M 60/851, F04B 43/00, F04B 43/04

(54) **DIAPHRAGM ASSEMBLY FOR A PULSATILE FLUID PUMP**
MEMBRANANORDNUNG FÜR EINE PULSIERENDE FLUIDPUMPE
ENSEMBLE DIAPHRAGME POUR POMPE À FLUIDE PULSATILE

(43) Date of publication of application: 03.01.2024
(73) Proprietor: Ventriflo, Inc., Pelham, NH 03076 (US)
(72) Inventor: VINCENT, Douglas E., Pelham, New Hampshire 03076 (US); KOENIG, George, Pelham, New Hampshire 03076 (US); POITRAS, James W., Pelham, New Hampshire 03076 (US); MURPHY, Matthew J., Pelham, New Hampshire 03076 (US)
(74) Representative: Potter Clarkson
(86) International application number: PCT/US2021/019253
(87) International publication number: WO 2022/182333

(56) References cited:
- CN-A- 113 048 040
- US-A- 2 954 738
- US-A- 5 092 879
- US-A- 5 349 896
- US-B2- 7 850 593

## Description

### Related Applications

The present application is one of four applications being filed on the same day and bearing attorney docket numbers 4747/1001, 4747/1002, 4747/1003, and 4747/1004.

### Technical Field

The present invention relates to pulsatile fluid pumps, and more particularly to pulsatile fluid pumps suitable for pumping blood.

### Background Art

A pulsatile fluid pump is taught in U.S. patent 7,850,593 ("our prior patent") for an invention of Douglas Vincent and Matthew Murphy, who are co-inventors of the present invention. Our prior patent discloses a pump actuated by a linear motor configured to cause reciprocation of a flexible membrane, serving as a wall of a fluid housing, that is in turn coupled to a pair of ball valves, in a manner as to implement pulsatile fluid flow.

### Summary of the Embodiments

A diaphragm assembly according to the present invention comprises the technical features as defined in independent claim 1.

In accordance with one embodiment of the invention, a diaphragm assembly for a pulsatile fluid pump includes an edge-mounted flexible diaphragm having an inside surface for contacting a fluid to be pumped, an outside surface exposed to ambient air, the diaphragm configured for operation cyclically between a diastole mode and a systole mode. The diaphragm assembly further includes a systolic distribution brace having a maximum radial extent and having an interior wall configured to cup a portion of the outside surface of the diaphragm, and a diastolic plate, embedded in the diaphragm, having a maximum radial extent and mechanically coupled to a portion of the inside surface of the diaphragm. The maximum radial extent of the systolic distribution brace at a periphery thereof is greater than the maximum radial extent of the diastolic plate. Further, the systolic distribution brace, the diastolic plate, and the diaphragm are mechanically linked to each other, so that: (i) in the course of the systole mode, the diaphragm overhangs the periphery of the systolic distribution brace and force is applied across the maximum radial extent of the systolic distribution brace, so as to impart tension in the diaphragm around the periphery of the systolic distribution brace; and (ii) in the course of the diastole mode, force is applied across the maximum radial extent of the diastolic plate, so as to impart tension in the diaphragm around the diastolic plate, wherein the differing radial extents of the systolic distribution brace and the diastolic plate produce differing distributions of force on the diaphragm over the course of these two modes, so as to distribute stress on the diaphragm and reduce strain on the diaphragm over the course of a pumping cycle.

Alternatively or in addition, the diaphragm has a reinforcing layer disposed between the systolic distribution brace and the diastolic plate.

Also alternatively or in addition, the systolic distribution brace has a rounded outer edge, and the diaphragm is configured to roll beyond the maximum radial extent of the systolic distribution brace and over the rounded outer edge.

Further alternatively or in addition, the interior wall of the systolic distribution brace that is configured to cup the portion of the outside surface of the diaphragm is disposed at an obtuse angle.

Also alternatively or in addition, the diastolic plate has a bevel. Alternatively or in addition, the diastolic plate has a bevel at an angle corresponding generally to the obtuse angle of the interior wall of the systolic distribution brace.

### Brief Description of the Drawings

The foregoing features of embodiments will be more readily understood by reference to the following detailed description, taken with reference to the accompanying drawings, in which:
Fig. **1** is a vertical section of an integral pump assembly **200** showing a diaphragm assembly **201** mounted to a pump-valving assembly **101** in diastole mode, in which the chamber **102** is being filled.
Fig. **2** is a vertical section of an integral pump assembly **200** showing a diaphragm assembly **201** mounted to a pump-valving assembly **101** in systole mode, in which the chamber **102** is being emptied.
Fig. **3** is an exploded perspective view, from the side, of an integral pump assembly **200** showing a pump-valving assembly **101,** diaphragm assembly **201,** and peripheral flange **221a.**
Fig. **4** is a vertical section of a diaphragm **202.**

### Detailed Description of Specific Embodiments

Definitions. As used in this description and the accompanying claims, the following terms shall have the meanings indicated, unless the context otherwise requires:
A "set" includes at least one member.
"Diastole mode" is a phase of operation of a pulsatile pump, according to embodiments of the present invention, during which the diaphragm **202** of the pump-valving assembly **101** is pulled away from the chamber **102** so as to create negative pressure within the chamber **102,** inlet ball check valve assembly **110,** and third tapered tract **126,** but not the fourth tapered tract **122**
"Systole mode" is a phase of operation of a pulsatile pump, according to embodiments of the present invention, during which the diaphragm **202** is pushed towards the chamber **102,** so as to create positive pressure within the chamber **102,** outlet ball check valve assembly **120,** and the second tapered tract **116,** but not the first tapered tract **112.**

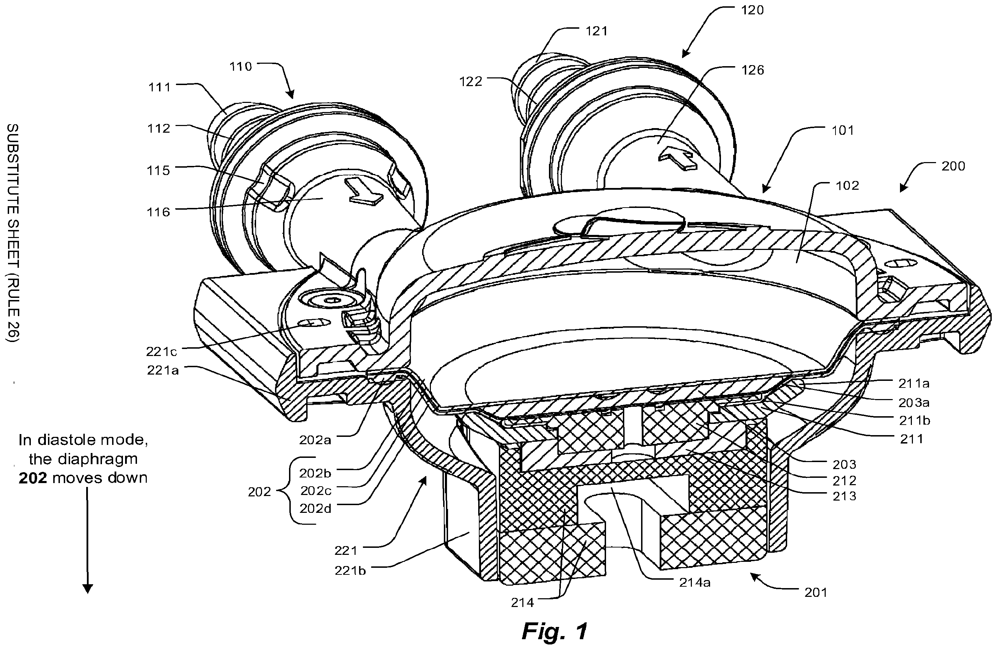

Fig. **1** is a vertical section of an integral pump assembly **200** showing a diaphragm assembly **201** mounted to a pump-valving assembly **101** in diastole mode, in which the chamber **102** is being filled. Among other things, the pump-valving assembly **101** includes an inlet ball check valve assembly **110** and an outlet ball check valve assembly **120.** The integral pump assembly **200** includes a pump housing **221** including a neck **221b** in which the coupler **214** reciprocates as the diaphragm **202** reciprocates. The neck **221b** is configured to maintain axial alignment of the coupler **214** and (via physical features, such as flattened sides) is also configured to maintain rotational alignment of the coupler **214.** The pump-valving assembly **101** is discussed in further detail in the related application, referenced above, bearing attorney docket number 4747/1001. As the diaphragm **202** is pulled down (in a direction away from the chamber **102**) to cause filling of the chamber **102,** it creates a negative pressure that draws fluid into the chamber **102** through the inlet port **111.** Similarly, as the diaphragm **202** is pushed up in a direction into the chamber **102,** it creates a positive pressure that causes flow of the fluid out of the chamber **102** through the outlet port **121.** (In these figures, like numbered items correspond to similar components across different figures.)

The edge mount **202a** diaphragm **202** (consisting of inside surface **202b,** reinforcing layer **202c,** and outside surface **202d**) is part of a diaphragm assembly **201** including, among other things, diastolic plate **203,** attachment stem **212,** systolic distribution brace **211,** and retaining ring **213.** The diastolic plate **203** is embedded within the diaphragm **202,** over the reinforcing layer **202c** and mechanically coupled to the inside surface **202b** of the diaphragm **202** and to the attachment stem **212.** As the diaphragm assembly **201** is pulled down to fill the chamber **102,** force is transmitted through the coupler **214** via the coupler slot **214a** to the attachment stem **212** to the diastolic plate **203,** which, in turn, exerts force on the reinforcing layer **202c** to minimize stress on the elastomeric inside surface **202b** and elastomeric outside surface **202d** of the diaphragm **202.** The attachment stem **212** is also coupled to the systolic distribution brace **211** through retaining ring **213.** The outer diameter of the diastolic plate **203** is smaller than the diameter of the interior of the rounded outer edge **211a** of the systolic distribution brace **211.** Construction of the diaphragm assembly **201** therefore is not symmetric for pulling and pushing. In pulling, through the attachment stem 212 and the diastolic plate **203,** stress is placed on a more concentrated portion of the diaphragm **202** (defined largely by the diameter of the diastolic plate **203**) than in pushing, which occurs more broadly through the attachment stem **212** and the systolic distribution brace **211,** which has a larger diameter than the diastolic plate **203** and is configured to cup the diaphragm **202.**

Through its expected useful life, the diaphragm **202** must tolerate millions of strokes. With each stroke, the diaphragm **202** is subjected to physical movement and oscillation between positive and negative pressures within the chamber **102.** In order to increase life expectancy, the ideal diaphragm **202** should be as thin as possible, because thin materials minimize bending stresses during flexure. A thin elastomeric material, used for the inside surface **202b** and outside surface **202d** of the diaphragm **202,** has excellent flexibility, but low tensile strength. This low tensile strength causes the elastomeric material to excessively strain during the oscillating positive and negative pressures seen during operation, eventually leading to failure. The reinforcing layer **202c** provides the tensile strength required to withstand these pressure fluctuations while maintaining the flexibility of the elastomeric layers **203** and **202d.**

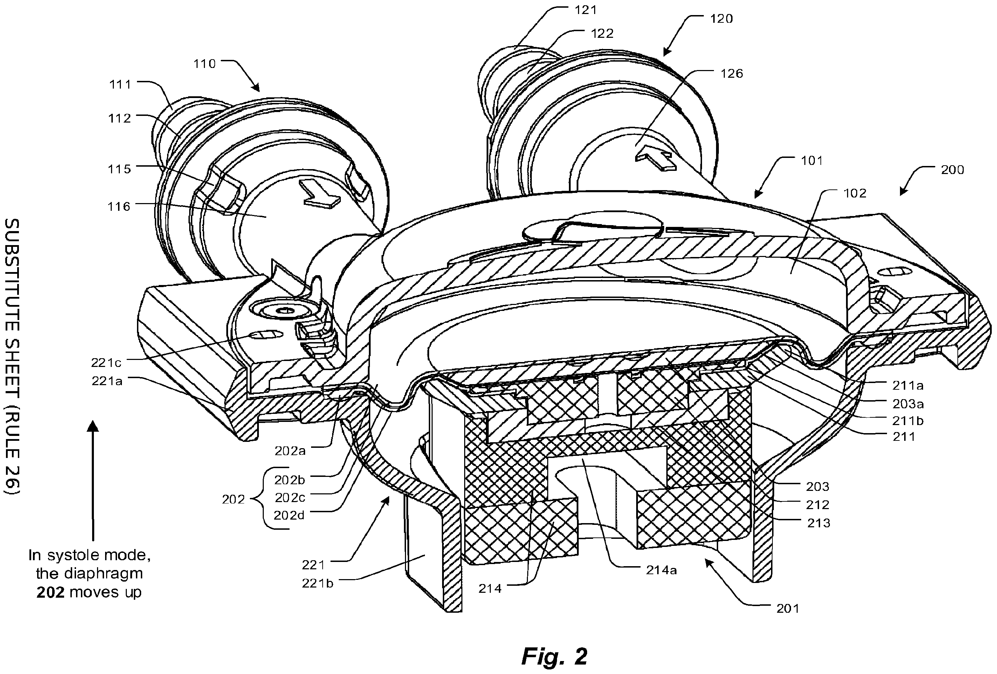

Fig. **2** is a vertical section of an integral pump assembly **200** showing a diaphragm assembly **201** mounted to a pump-valving assembly **101** in systole mode, in which the chamber **102** is being emptied. The systolic distribution brace **211** is mounted beneath the diaphragm **202** and mechanically coupled to the diastolic plate **203** through the attachment stem **212.** As the diaphragm assembly **201** is pushed up to empty the chamber **102,** force is transmitted through the coupler **214** via the coupler slot **214a** to both the retaining ring **213** (and on to the systolic distribution brace **211**) and to the systolic distribution brace **211** directly, which systolic distribution brace **211** cups and exerts force on the diaphragm **202** (including the reinforcing layer **202c**)**,** distributing the stress over a changing area of the diaphragm **202** as it wraps around the rounded outer edge **211a** of the systolic distribution brace **211,** reducing strain on the diaphragm **202** over the course of a pumping cycle, and thereby increasing its life.

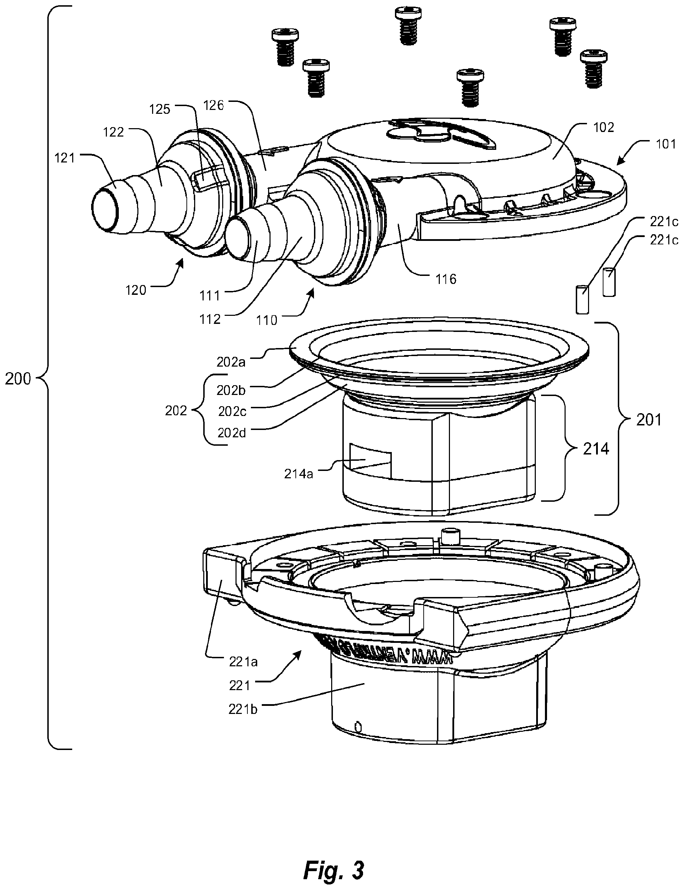

Fig. **3** is an exploded perspective view, from the side, of an integral pump assembly **200,** showing a pump-valving assembly **101,** chamber **102,** inlet port **111,** first tapered tract **112,** second tapered tract **116,** outlet port **121,** fourth tapered tract **121,** outlet rib **125,** third tapered tract **126,** diaphragm assembly **201,** edge mount **202a** diaphragm **202** (composed of inside surface **202b,** reinforcing layer **202c,** and outside surface **202d**), pump housing **221** (with peripheral flange **221a,** neck **221b,** and compliant member **221c.** Items **101-126** are discussed in further detail in the related application, referenced above, bearing attorney docket number 4747/1001.

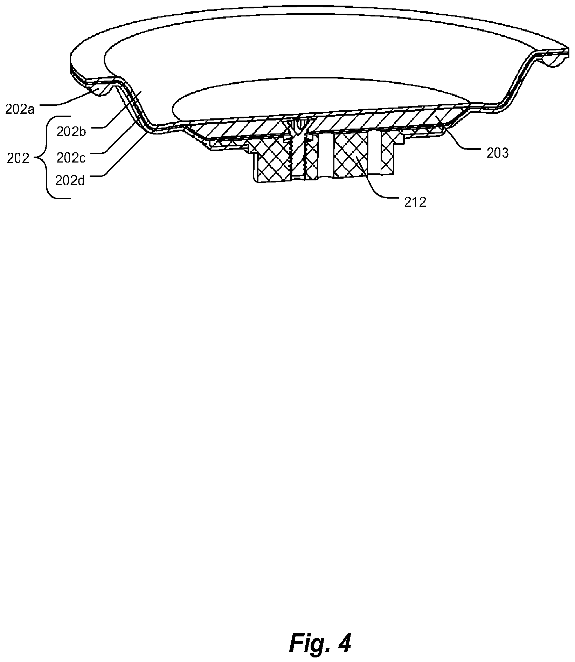

Fig. **4** is a vertical section of a diaphragm **202** (composed of edge mount **202a,** inside surface **202b,** reinforcing layer **202c,** and outside surface **202d**), also showing the diastolic plate **203** and attachment stem **212.**

The structure of a pulsatile pump in accordance with various embodiments of the present invention can usefully reflect attributes of the human heart. The human heart is preload sensitive-the heart cannot "pull" blood into the left ventricle; it can only allow the blood available to flow naturally into the ventricle. The human heart is also afterload sensitive in that it is responsive to the compliance and resistance in the downstream vasculature and doesn't exert excess force on the blood, which could damage the vasculature. Lastly, the left ventricle cannot deliver blood that isn't in the ventricle when it contracts; there is a limited bolus of blood that it can deliver.

The pump-valving assembly **101** has similar attributes of inherent safety: it is preload and afterload sensitive, and it is limited in both the volume of blood it can deliver and the force at which it can deliver that bolus of blood. When filling, the pump-valving assembly **101** allows gravity filling from the venous reservoir, exerting minimal negative pressure. When the chamber in the pump-valving assembly **101** is emptying, the linear motor that powers the pump valving assembly is limited by design. In consequence the pump valving assembly cannot overpressure the downstream tubing or vasculature, instead delivering less than the volume of blood in the pump chamber **102,** thereby only delivering as much volume as the vasculature can receive.

The pump-valving assembly **101** is analogous to a left ventricle of the human heart; the inlet ball check valve assembly **110** used in various embodiments hereof is analogous to a mitral valve; and the outlet ball check valve assembly **120** used in various embodiments hereto is analogous to an aortic valve. Like the human heart, the inlet **110** and outlet **120** ball check valve assemblies are passive and require a slight reversal of flow to close. This slight reversal of flow mimics the slight reversal that occurs when the aortic valve of the human heart closes.

The embodiments of the invention described above are intended to be merely exemplary and the invention is defined by the appended claims.

## Claims

1. A diaphragm assembly (201) for a pulsatile fluid pump comprising
an edge-mount flexible diaphragm (202) having an inside surface (202b) for contacting a fluid to be pumped, an outside surface (202d) exposed to ambient air, the diaphragm configured for operation cyclically between a diastole mode and a systole mode;
a systolic distribution brace (211) having a maximum radial extent and having an interior wall configured to cup a portion of the outside surface of the diaphragm;
a diastolic plate (203), embedded in the diaphragm, having a maximum radial extent and mechanically coupled to a portion of the inside surface of the diaphragm;
wherein the maximum radial extent of the systolic distribution brace at a periphery thereof is greater than the maximum radial extent of the diastolic plate, and the systolic distribution brace, the diastolic plate, and the diaphragm are mechanically linked to each other, so that:
(i) in the course of the systole mode, the diaphragm overhangs the periphery of the systolic distribution brace and force is applied across the maximum radial extent of the systolic distribution brace, so as to impart tension in the diaphragm around the periphery of the systolic distribution brace, and
(ii) in the course of the diastole mode, force is applied across the maximum radial extent of the diastolic plate, so as to impart tension in the diaphragm around the diastolic plate, wherein the differing radial extents of the systolic distribution brace and the diastolic plate produce differing distributions of force on the diaphragm over the course of these two modes, so as to distribute stress on the diaphragm and reduce strain on the diaphragm over the course of a pumping cycle.

2. A diaphragm assembly according to claim 1, wherein the diaphragm has a reinforcing layer (202c) disposed between the systolic distribution brace and the diastolic plate.

3. A diaphragm according to claim 1, wherein the systolic distribution brace has a rounded outer edge (211a) and the diaphragm is configured to roll beyond the maximum radial extent of the systolic distribution brace and over the rounded outer edge.

4. A diaphragm assembly according to claim **1,** wherein the interior wall of the systolic distribution brace that is configured to cup the portion of the outside surface of the diaphragm is disposed at an obtuse angle.

5. A diaphragm assembly according to claim **1,** wherein diastolic plate has a bevel.

6. A diaphragm assembly according to claim **1,** wherein diastolic plate has a bevel at an angle corresponding generally to the obtuse angle of the interior wall of the systolic distribution brace.

## Patentansprüche

1. Membranbaugruppe (201) für eine pulsierende Fluidpumpe, umfassend:
eine an der Kante montierte flexible Membran (202), die eine innere Oberfläche (202b) zum Kontaktieren eines Fluids aufweist, um gepumpt zu werden, eine äußere Oberfläche (202d), die der Umgebungsluft ausgesetzt ist, wobei die Membran konfiguriert ist, zyklisch zwischen einem Diastolenmodus und einem Systolenmodus zu operieren;
eine systolische Verteilungsabstützung (211), die einen maximalen Radialumfang und eine innere Wand aufweist, die konfiguriert ist, einen Abschnitt der äußeren Oberfläche der Membran zu umschließen;
eine diastolische Platte (203), die in der Membran eingebettet ist, einen maximalen Radialumfang aufweist und mechanisch mit einem Abschnitt der inneren Oberfläche der Membran gekoppelt ist;
wobei der maximale Radialumfang der systolischen Verteilungsabstützung an einer Peripherie davon größer ist als der maximale Radialumfang der diastolischen Platte, und die systolische Verteilungsabstützung, die diastolische Platte und die Membran mechanisch miteinander verbunden sind, sodass:
(i) im Verlauf des Systolenmodus die Membran die Peripherie der systolischen Verteilungsabstützung überhängt und eine Kraft über den maximalen Radialumfang der systolischen Verteilungsabstützung angewendet wird, sodass Spannung auf die Membran um die Peripherie der systolischen Verteilungsabstützung erzeugt wird, und
(ii) im Verlauf des Diastolenmodus eine Kraft über den maximalen Radialumfang der diastolischen Platte angewendet wird, sodass Spannung auf die Membran um die diastolische Platte erzeugt wird, wobei die verschiedenen Radialumfänge der systolischen Verteilungsabstützung und der diastolischen Platte verschiedene Verteilungen der Kraft auf die Membran im Verlauf dieser zwei Modi bewirken, sodass Belastung auf die Membran verteilt und Dehnung auf die Membran im Verlauf eines Pumpzyklus verringert wird.

2. Membranbaugruppe nach Anspruch **1,** wobei die Membran eine Verstärkungsschicht (202c) aufweist, die zwischen der systolischen Verteilungsabstützung und der diastolischen Platte angeordnet ist

3. Membran nach Anspruch **1,** wobei die systolische Verteilungsabstützung eine abgerundete äußere Kante (211a) aufweist
und die Membran konfiguriert ist, über den maximalen Radialumfang der systolischen Verteilungsabstützung und über die abgerundete äußere Kante zu rollen.

4. Membranbaugruppe nach Anspruch **1,** wobei die innere Wand der systolischen Verteilungsabstützung, die konfiguriert ist, den Abschnitt der äußeren Oberfläche der Membran zu umschließen, in einem stumpfen Winkel angeordnet ist.

5. Membranbaugruppe nach Anspruch **1,** wobei die diastolische Platte eine Abschrägung aufweist.

6. Membranbaugruppe nach Anspruch **1,** wobei die diastolische Platte eine Abschrägung in einem Winkel aufweist, der im Allgemeinen dem stumpfen Winkel der inneren Wand der systolischen Verteilungsabstützung entspricht.

## Revendications

1. Ensemble diaphragme (201) pour une pompe à fluide pulsatile comprenant :
un diaphragme flexible monté sur le bord (202) ayant une surface intérieure (202b) destinée à entrer en contact avec un fluide devant être pompé, une surface extérieure (202d) exposée à l'air ambiant, le diaphragme étant configuré pour une opération cyclique entre un mode diastole et un mode systole ;
une entretoise de distribution systolique (211) ayant une étendue radiale maximale et ayant une paroi intérieure configurée pour englober une partie de la surface extérieure du diaphragme ;
une plaque diastolique (203), intégrée dans le diaphragme, ayant une étendue radiale maximale et mécaniquement accouplée à une partie de la surface intérieure du diaphragme ;
dans lequel l'étendue radiale maximale de l'entretoise de distribution systolique au niveau d'une périphérie de celle-ci est supérieure à l'étendue radiale maximale de la plaque diastolique, et l'entretoise de distribution systolique, la plaque diastolique, et le diaphragme sont mécaniquement reliés les uns aux autres, de sorte que :
(i) au cours du mode systole, le diaphragme surplombe la périphérie de l'entretoise de distribution systolique et une force est appliquée sur l'ensemble de l'étendue radiale maximale de l'entretoise de distribution systolique, de manière à conférer une tension dans le diaphragme autour de la périphérie de l'entretoise de distribution systolique, et
(ii) au cours du mode diastole, une force est appliquée sur l'ensemble de l'étendue radiale maximale de la plaque diastolique, de manière à conférer une tension dans le diaphragme autour de la plaque diastolique, dans lequel les étendues radiales différentes de l'entretoise de distribution systolique et de la plaque diastolique produisent des distributions de force différentes sur le diaphragme au cours de ces deux modes, de manière à distribuer une pression sur le diaphragme et à réduire une contrainte sur le diaphragme au cours d'un cycle de pompage.

2. Ensemble diaphragme selon la revendication 1, dans lequel le diaphragme présente une couche de renforcement (202c) disposée entre l'entretoise de distribution systolique et la plaque diastolique.

3. Diaphragme selon la revendication 1, dans lequel l'entretoise de distribution systolique présente un bord externe arrondi (211a) et le diaphragme est configuré pour rouler au-delà de l'étendue radiale maximale de l'entretoise de distribution systolique et sur le bord externe arrondi.

4. Ensemble diaphragme selon la revendication 1, dans lequel la paroi intérieure de l'entretoise de distribution systolique qui est configurée pour englober la partie de la surface extérieure du diaphragme est disposée selon un angle obtus.

5. Ensemble diaphragme selon la revendication 1, dans lequel la plaque diastolique présente un biseau.

6. Ensemble diaphragme selon la revendication 1, dans lequel la plaque diastolique présente un biseau selon un angle correspondant généralement à l'angle obtus de la paroi intérieure de l'entretoise de distribution systolique.
